# EUROPEAN PATENT APPLICATION

(11) **EP 4 082 475 A1**
(43) Date of publication of application: **02.11.2022**
(21) Application number: 20905715.7
(22) Date of filing: 22.12.2020
(51) Int. Cl.: A61C 7/36, A61C 19/00, A61M 16/06, A61F 5/56

(54) **MOUTHPIECE AND METHOD FOR MANUFACTURING SAME**

(30) Priority: 23.12.2019 JP 2019231821
(71) Applicant: Mitsui Chemicals, Inc., Tokyo 105-7122 (JP)
(72) Inventor: YUKITA, Takashi, Sodegaura-shi, Chiba 299-0265 (JP); IWABUCHI, Yuki, Sodegaura-shi, Chiba 299-0265 (JP); HASEGAWA, Akira, Sodegaura-shi, Chiba 299-0265 (JP)
(74) Representative: Epping - Hermann - Fischer
(86) International application number: PCT/JP2020/048026
(87) International publication number: WO 2021/132274

(57) **Abstract**

The present invention addresses the problem of providing: a mouthpiece having a shape that adequately conforms to the teeth rows, etc., of a user, the mouthpiece having an excellent feel during wearing; and a method for manufacturing the mouthpiece. A mouthpiece for solving the abovementioned problem includes a mouthpiece body part having a hard section, which has a groove for accommodating the teeth rows and/or the gums, and a soft section, which contains a material having a lower bending elasticity than that of the hard section. The proportion of the maximum thickness of the soft section to the minimum thickness of the soft section is 5 or greater.

## Description

### Technical Field

The present invention relates to an oral appliance and a method for manufacturing the same.

### Background Art

Obstructive sleep apnea is considered to be mainly caused by blockage of the airway due to dropping of the lower jaw. Accordingly, various oral appliances have been suggested to remedy obstructive sleep apnea. These oral appliances remedy apnea and snoring during sleep by restricting the backward displacement of the lower jaw of a user and thereby keeping the airway wide open.

For example, Patent Literature (hereinafter, abbreviated as PTL) 1 suggests an oral appliance having a pair of oral appliance body sections corresponding to the upper jaw and the lower jaw and a connecting member made of metal connecting these oral appliance bodies. The oral appliance adjusts the relative position of the upper jaw oral appliance body section and the lower jaw oral appliance body section by the connecting member, and displaces the lower jaw of the user wearing the oral appliance toward the forward direction. In the oral appliance, a connecting member made of metal is fixed on an oral appliance body section made of resin.

Since such an oral appliance is held in a state where the position of the lower jaw is displaced, a material having a high hardness is used in the oral appliance body section. However, when an oral appliance is constituted only of a material having a high hardness, it is difficult to closely adhere an oral appliance body section to a dentition and the like, so that sufficient fixation cannot be achieved. Consequently, the oral appliance has been fixed by disposing a protrusion made of metal on a surface of the oral appliance body section opposite to the dentition and engaging the protrusion and the dentition and the like.

However, the oral appliance having a high hardness of the oral appliance body section has a poor comfort of wearing. Accordingly, it is suggested to provide a soft part inside of the oral appliance body section, that is, on a side facing the dentition and the like (PTL 2).

### Citation List

### Patent Literature

PTL1
   U.S. Patent No. 2007/0224567
PTL2
   WO2017/154641

### Summary of Invention

### Technical Problem

In the oral appliance of the above PTL 2, the soft part having a relatively low hardness is disposed on the dentition side. Thus, the comfort of wearing tends to be better than the conventional oral appliances. With this constitution, the oral appliance body section is more likely to closely adhere the dentition and the like, so that the oral appliance can be fixed on the upper jaw or the lower jaw without providing a protrusion inside the oral appliance body section. However, when a dentition and the like have a complex shape, a gap tends to occur between the soft part and the dentition and the like. The occurrence of such a gap leads to the problems of lack of comfort of wearing of the oral appliance and dropping of the oral appliance from the upper jaw or the lower jaw.

The present invention has been made in view of the above problems. Specifically, an object of the present invention is to provide an oral appliance having a shape sufficiently corresponding to the dentition and the like of a user and a good comfort of wearing, and a method for manufacturing the same.

### Solution to Problem

To solve the above problems, the present invention provides the following oral appliance.

An oral appliance comprising an oral appliance body section including a hard part having a groove for accommodating a dentition and/or gums, and a soft part disposed on the inner wall of the groove, the soft part comprising a material having a flexural modulus lower than that of the hard part, wherein a ratio of a maximum thickness of the soft part to a minimum thickness of the soft part is 5 or more.

The present invention also provides a method for manufacturing the following oral appliance.

A method for manufacturing an oral appliance comprising: preparing a hard part having a groove for accommodating a dentition and/or gums; injecting a curable resin composition into the groove of the hard part; disposing a model having a shape of a dentition and/or gums in the groove of the hard part; and forming a soft part comprising a material having a flexural modulus lower than that of the hard part by curing the curable resin composition in a state where the curable resin composition and the model are in contact with each other.

### Advantageous Effects of Invention

The oral appliance of the present invention has a shape sufficiently corresponding to the dentition and the like of a user and a good comfort of wearing. In addition, according to the manufacturing method of the present invention, the oral appliance can be manufactured accurately.

### Brief Description of Drawings

FIG. 1 is a schematic front view of an oral appliance according to an embodiment of the present invention;
FIG. 2A is a schematic side view of the oral appliance illustrated in FIG. 1 when a mouth is closed, and FIG. 2B is a schematic side view of the oral appliance illustrated in FIG. 1 when a mouth is opened;
FIG. 3A is a cross-sectional view along line A-A of the oral appliance body section of the oral appliance illustrated in FIG. 1, and FIG. 3B is a plan view of the upper jaw oral appliance body section of the oral appliance illustrated in FIG. 1;
FIGS. 4A to 4C are diagrams illustrating one example of a method for producing a hard part of the oral appliance body section of the oral appliance illustrated in FIG. 1; and
FIGS. 5A to 5C are diagrams illustrating one example of a method for producing a soft part of the oral appliance body section of the oral appliance illustrated in FIG. 1.

### Description of Embodiments

Hereinafter, the oral appliance of the present invention will be described based on an embodiment. However, the oral appliance of the present invention is not limited to this embodiment. In the following description, "forward" or "front" and "backward" or "rear" respectively mean a direction toward the front of the user wearing the oral appliance (a direction toward the lip side, as viewed from the tongue body in the oral cavity) and a direction toward the rear of the user (a direction toward the throat side, as viewed from the tongue body in the oral cavity). In addition, "downward" means a direction towards a lower place of the user wearing the oral appliance (a direction from the head towards the feet of the user when the user stands erect), and "lateral direction" means a direction toward the left and right facing the front of the user on the basis of the middle of the upper jaw of the user wearing the oral appliance (specifically, a direction towards both cheeks, as viewed from the middle of the upper jaw). The "outside" and the "inside" respectively mean a side close to the body surface and a side far from the body surface of the user wearing the oral appliance.

In the following description, a state where the mouth of the user wearing the oral appliance is closed is referred to as "when a mouth is closed", and a state where the mouth of the user wearing the oral appliance is opened is referred to as "when a mouth is opened". When a mouth is closed, the upper jaw oral appliance and the lower jaw oral appliance are disposed so that their opposing surfaces can be substantially parallel to each other, and when a mouth is opened, the lower jaw oral appliance moves on an arc-shaped track, and thus the upper jaw oral appliance and the lower jaw oral appliance are disposed non-parallel to each other so that their opposing surfaces can have an angle of about 45° at most.

### 1. Oral Appliance of Present Embodiment

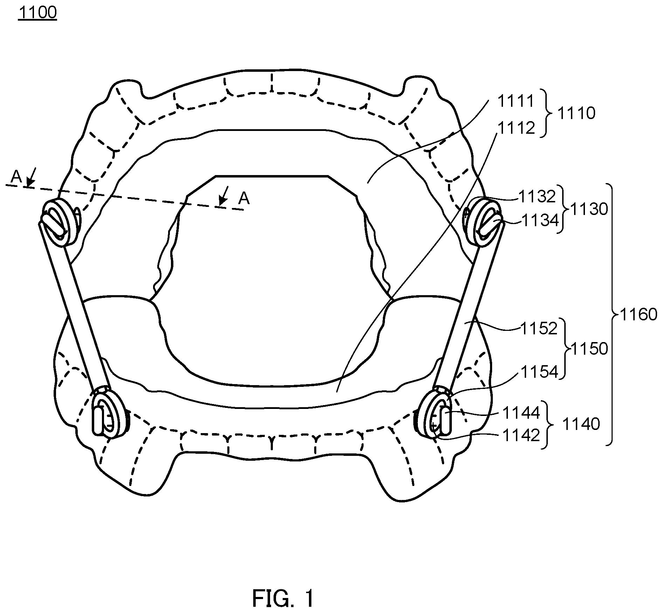

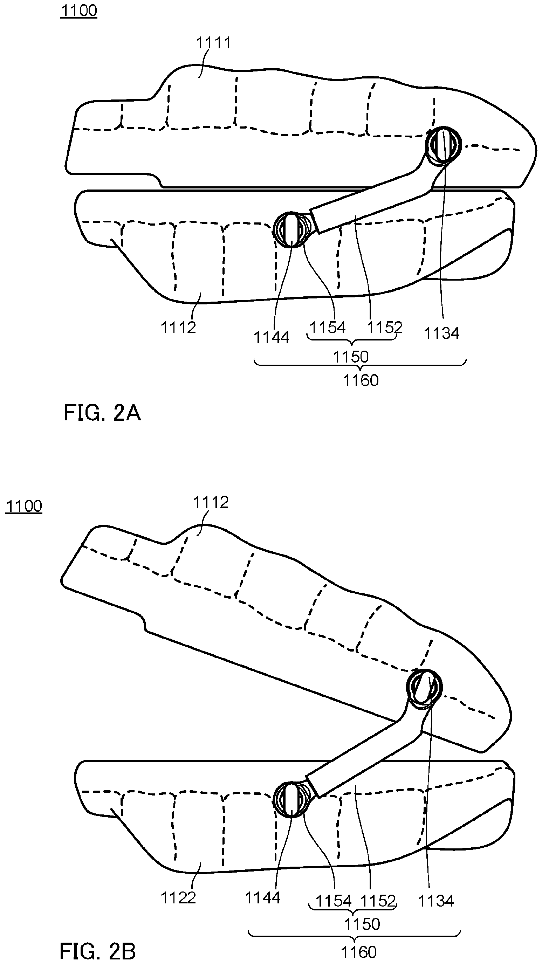

FIG. 1 is a schematic front view of oral appliance 1100 according to an embodiment of the present invention, FIG. 2A is a schematic side view of oral appliance 1100 when a mouth is closed, and FIG. 2B is a schematic side view of oral appliance 1100 when a mouth is opened.

As illustrated in FIG. 1, FIG. 2A, and FIG. 2B, oral appliance 1100 of the present embodiment has a pair of oral appliance body sections 1110 which consist of upper jaw oral appliance body section 1111 and lower jaw oral appliance body section 1112, and a pair of left and right positioning members 1160 for restricting the backward displacement of lower jaw oral appliance body section 1112.

In oral appliance 1100 of the present embodiment, a relative position of upper jaw oral appliance body section 1111 and lower jaw oral appliance body section 1112 is determined by positioning member 1160, and the position of the lower jaw with respect to the upper jaw of the user is suitably adjusted. Thus, the airway is kept wide open during sleep, so that apnea and snoring are remedied.

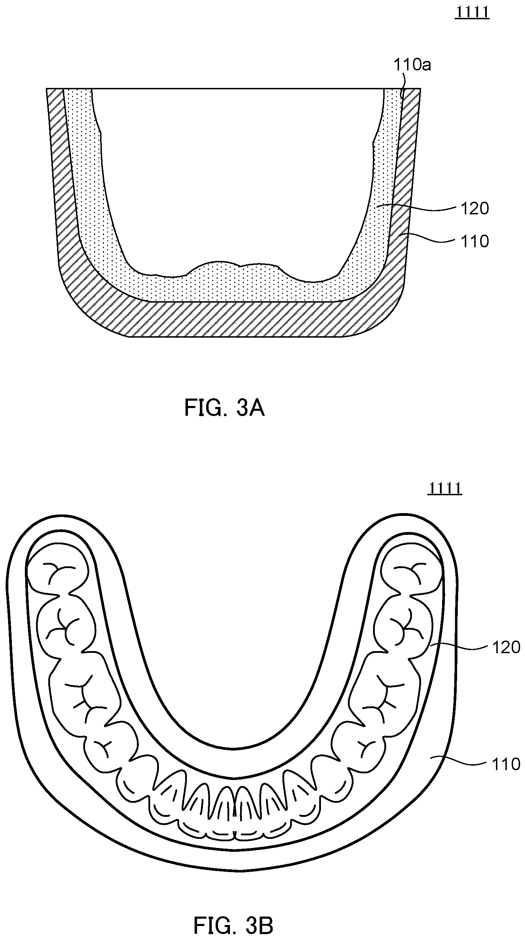

FIG. 3A is a cross-sectional view along line A-A of upper jaw oral appliance body section 1111 of oral appliance 1100 in FIG. 1, and FIG. 3B is a plan view of upper jaw oral appliance body section 1111. As illustrated in FIG. 3A and FIG. 3B, oral appliance body section 1110 of the present invention (in FIG. 3A, upper jaw oral appliance body section 1111) has a two-layer structure. As illustrated in FIG. 3A and FIG. 3B, upper jaw oral appliance body section 1111 has hard part 110 having groove 110a, and soft part 120 that is disposed on the wall surface and the bottom surface of groove 110a of hard part 110 and corresponds to the shape of the dentition, gums, and the like of the user. The user wears oral appliance 1100 so that the dentition and the gums can closely adhere to soft part 120. Lower jaw oral appliance body section 1112 also has a similar two-layer structure. In oral appliance 1100 which has upper jaw oral appliance body section 1111 and lower jaw oral appliance body section 1112 having such a two-layer structure (hereinafter, when there is no need to distinguish upper jaw oral appliance body section 1111 and lower jaw oral appliance body section 1112, they are collectively referred to as "oral appliance body section 1110"), soft part 120 having a relatively low elasticity comes into contact with the dentition and the gums, so that the comfort of wearing is good.

As mentioned above, it is also suggested in conventional oral appliances to make an oral appliance body section have a two-layer structure of the hard part and the soft part. However, in conventional oral appliances, it is difficult to make the soft part correspond to the complex shape of the dentition and the like, and there has been a case where a gap is formed between the soft part and the dentition or the gums. This is considered to be due to the manufacturing method of the conventional oral appliance body section having a two-layer structure.

In the conventional oral appliance body section, first, a laminated sheet having a two-layer structure that includes a hard layer for forming the hard part and a soft layer for forming the soft part is prepared. Then, the laminated sheet is softened by heat and the like, and a model having a shape of the dentition and the like is pressed against the soft layer side. The shape of the dentition and the like is thus transferred to the soft sheet, so that an oral appliance body section having the desired form is obtained. However, since the original soft layer has a uniform thickness by this method, the deformation amount of the soft layer is limited. Thus, it is difficult to allow the soft layer to sufficiently trace the model of a complex structure and the like. In particular, when the model has unevenness greater than the thickness of the soft layer, a gap tends to be generated between the model and the soft layer.

In contrast, in oral appliance 1100 of the present embodiment, the ratio of the maximum thickness of soft part 120 to the minimum thickness of soft part 120 is 5 or more. That is, the difference between the minimum thickness of soft part 120 and the maximum thickness of the soft part is large, and thus oral appliance 1100 of the present embodiment can sufficiently correspond to the large unevenness to which the soft part of the conventional oral appliances cannot correspond. Therefore, oral appliance 1100 has a good comfort of wearing, and sufficiently, closely adhere to the dentition and the gums when being worn in the oral cavity.

Here, as a method for producing oral appliance 1100 having a large ratio of the maximum thickness of soft part 120 to the minimum thickness of soft part 120 like the present embodiment, a method in which hard part 110 and soft part 120 are separately produced and then bonded together is considered. However, when only soft part 120 is produced by closely adhering a resin for forming the soft part to a dentition model, it is difficult to peel soft part 120 from the dentition model and desired oral appliance 1100 (soft part 120) cannot be obtained. In addition, when the thickness of soft part 120 is thin, the above peeling is particularly difficult. In contrast, in oral appliance 1100 of the present embodiment, a liquid curable resin composition is disposed between the hard layer and the model, and the curable resin composition is cured, whereby the soft layer is formed, as described below. Thus, even when the model has a complex shape, the curable resin composition can sufficiently trace the model, so that soft layer 120 having a thickness ratio as mentioned above is obtained.

Hereinafter, each constitution of the oral appliance of the present embodiment will be described, and thereafter, the manufacturing method will be described.

### (Oral appliance body section)

As mentioned above, oral appliance body section 1110 of oral appliance 1100 of the present embodiment is constituted from upper jaw oral appliance body section 1111 for wearing in the upper jaw and lower jaw oral appliance body section 1112 for wearing in the lower jaw. These oral appliance bodies are connected by positioning member 1160 described below.

In FIG. 1, oral appliance body section 1110 is formed so as to cover all upper and lower dentitions of the user, but oral appliance body section 1110 may not necessarily cover all dentitions. For example, oral appliance body section 1110 may cover only a part of the dentition or the gums, or may not cover the incisor. In this case, it is preferable that structures covering left and right molars be connected with each other by a wire and the like disposed along the dentition. However, to increase the strength of oral appliance body section 1110 or the comfort of wearing, a structure covering all upper and lower dentitions, as illustrated in FIG. 1, is preferable.

Here, each of upper jaw oral appliance body section 1111 and lower jaw oral appliance body section 1112 has a two-layer structure of hard part 110 and soft part 120.

### • Hard Part

Hard part 110 is a member having a relatively high hardness which is disposed on the outside (buccal side) and the inside (tongue side) of oral appliance body section 1110 and on an area where upper jaw oral appliance body section 1111 and lower jaw oral appliance body section 1112 are opposed to each other.

The outer shape of hard part 110 is not particularly limited, and is appropriately selected in accordance with the shape in the oral cavity of the user. The outer shape may be constituted from a smooth plane or a smooth curved plane, and unevenness may be formed in accordance with the shape of the dentition and the like of the user.

The hard part 110 has groove 110a for accommodating the dentition and the like. The width of groove 110a is appropriately set in accordance with the width of the dentition and the gums to be accommodated, and is only required to be larger than the width of the dentition and the gums to be accommodated. On the other hand, the depth of groove 110a is also appropriately set in accordance with the shape of the dentition and the gums to be accommodated. Here, the wall surface and the bottom surface of groove 110a may be constituted from a smooth plane or a curved plane, and unevenness may be formed in accordance with the shape of the dentition and the like of the user. The thickness of hard part 110 may be constant or different.

Here, the flexural modulus of hard part 110 is preferably 50 to 4,000 MPa, more preferably 50 to 3,000 MPa, and further preferably 200 to 3,000 MPa. The flexural modulus is measured according to JIS T6501. Particularly when the flexural modulus of the hard part is 1,000 MPa or more, the strength of oral appliance body section 1110 is particularly high.

On the other hand, the tensile strength of hard part 110 is preferably 10 N or more and 1,000 N or less, and more preferably 100 to 800 N. When the tensile strength of hard part 110 falls within the range, the tracing property of hard part 110 with respect to the dentition and the like tends to be increased. The tensile strength means the strength at which hard part 110 is ruptured, when a hole of φ 1.5 mm is opened in a position corresponding to teeth number 6 in hard part 110 of oral appliance produced using a standard model manufactured by Nissin Dental Products INC (thickness of 3 mm), and a tensile test in the molar direction (backward) is performed.

Further, the density of the material constituting hard part 110 is preferably less than 1.5 kg/m³, more preferably 0.8 to 1.4 kg/m³, and still more preferably 0.8 to 1.3 kg/m³. When the density of the material constituting hard part 110 is less than 1.5 kg/m³, the weight of oral appliance 1100 can be reduced and usability tends to be good.

The material of hard part 110 may be metal, resin, or a composite of resin and metal. Hard part 110 may further contain a filler, various additives, or the like within a range of not impairing the object and effect of the present invention.

The metal contained in hard part 110 is only required to be a metal that hardly affects the human body, and examples thereof include titanium, iron (such as steel including stainless steel), gold, silver, platinum, cobalt, chrome, and alloys thereof. These metals may have increased corrosion resistance by chemical conversion coating or the like.

On the other hand, the resin contained in hard part 110 is only required to have a desired hardness (for example, the above modulus), and examples thereof include a (meth)acrylic resin. As used herein, "(meth)acrylic" refers to acrylic or methacrylic, or both of them.

The (meth)acrylic resin is only required to be a resin mainly including a structural unit derived from a monofunctional or polyfunctional (meth)acrylic monomer. The structural unit derived from the (meth)acrylic monomer is preferably included in an amount of 50 mass% to 100 mass%, more preferably in an amount of 60 mass% to 100 mass%, and particularly preferably in an amount of 80 mass% to 100 mass% based on the total amount of the structural unit of the (meth)acrylic resin.

Here, the above (meth)acrylic resin particularly preferably includes a structure derived from at least one (meth)acrylic monomer selected from the group consisting of (A) a di(meth)acrylic monomer having no aromatic ring and having one or more ether bonds and two (meth)acryloyloxy groups in one molecule, (B) a (meth)acrylic monomer having no aromatic ring and having a ring structure other than the aromatic ring and one (meth)acryloyloxy group in one molecule, (C) a di(meth)acrylic monomer having neither aromatic ring nor ether bond and having a hydrocarbon skeleton and two (meth)acryloyloxy groups in one molecule, and (D) a (meth)acrylic monomer having at least one aromatic ring and one (meth)acryloyloxy group in one molecule.

Examples of (A) the di(meth)acrylic monomer described above include a compound represented by the following general formula (a-1) or (a-2): 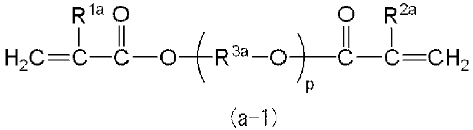 wherein each of R^{1a} and R^{2a} independently represents a hydrogen atom or a methyl group; R^{3a} represents a linear or branched alkylene group having 2 to 4 carbon atoms; and p represents 2 to 4, 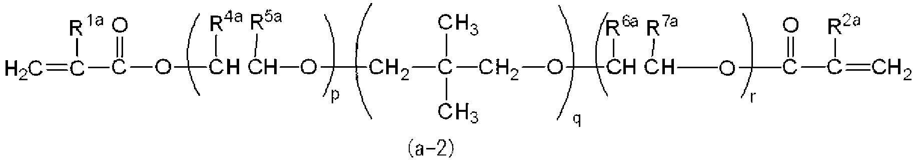 wherein each of R^{1a}, R^{2a}, R^{4a}, R^{5a}, R^{6a}, and R^{7a} independently represents a hydrogen atom or a methyl group; each of p, q, and r independently represents 0 or 1, provided that p + q + r ≥ 2 is satisfied.

Examples of (B) the (meth)acrylic monomer described above include a compound represented by the following general formula (b-1) or (b-2): 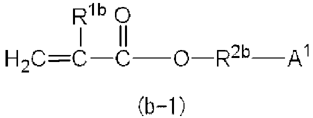 wherein R^{1b} represents a hydrogen atom or a methyl group; R^{2b} represents a single bond or a methylene group; and A¹ represents a ring structure other than the aromatic ring, 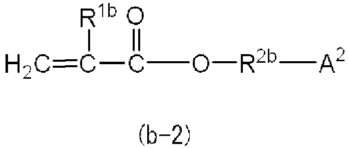 wherein R^{1b} represents a hydrogen atom or a methyl group; R^{2b} represents a single bond or a methylene group; and A² represents a ring structure having a dicyclopentenyl skeleton, a dicyclopentanyl skeleton, a cyclohexane skeleton, a tetrahydrofuran skeleton, a morpholine skeleton, an isobornyl skeleton, a norbornyl skeleton, a dioxolane skeleton, or a dioxane skeleton.

Examples of (C) the di(meth)acrylic monomer include a compound represented by the following general formula (c-1) or (c-2): 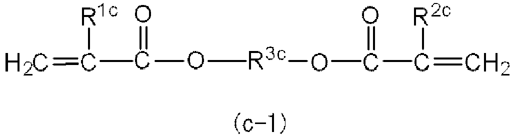 wherein each of R^{1c} and R^{2c} independently represents a hydrogen atom or a methyl group; and R^{3c} represents an alkylene group having 1 to 9 carbon atoms, 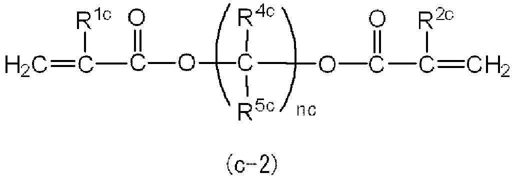 wherein each of R^{1c} and R^{2c} independently represents a hydrogen atom or a methyl group; each of R^{4c} and R^{5c} independently represents a hydrogen atom or a methyl group; and nc represents 1 to 9, provided that the number of carbon atoms of the alkylene group represented by -(CR^{4c}R^{5c})_{nc}- is 1 to 9.

Examples of (D) the (meth)acrylic monomer include a compound represented by the following general formula (d-1) or (d-2): 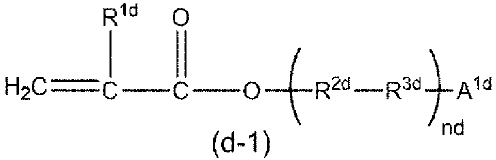 wherein R^{1d} represents a hydrogen atom or a methyl group; R^{2d} represents a single bond or a linear or branched alkylene group having 1 to 5 carbon atoms; R^{3d} represents a single bond, an ether bond (-O-), an ester bond (-O-(C=O)-), or -C₆H₄-O-; A^{1d} represents at least one aromatic ring optionally having a substituent; and nd represents 1 to 2, 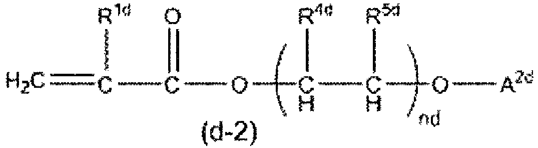 wherein each of R^{1d}, R^{4d}, and R^{5d} independently represents a hydrogen atom or a methyl group; A^{2d} represents at least one aromatic ring optionally having a substituent; and nd represents 1 to 2.

The (meth)acrylic resin may include a structural unit derived from a monomer other than the (meth)acrylic monomer within a range not impairing the object and effect of the present invention. Examples of the monomer other than the (meth)acrylic monomer include (meth)acrylic acid (preferably methacrylic acid) and α-olefin (preferably ethylene, propylene, or butylene).

Here, the weight average molecular weight (Mw) of the above (meth)acrylic resin is preferably 10,000 to 1,000,000, more preferably 20,000 to 800,000, and particularly preferably 50,000 to 500,000. The weight average molecular weight (Mw) is a value measured by gel permeation chromatography (GPC), and a value in terms of polymethyl methacrylate. When the weight average molecular weight (Mw) of (meth)acrylic resin falls within the above range, a hard part having a high strength can be obtained.

### • Soft Part

Soft part 120 is a member disposed inside groove 110a of hard part 110, and has a shape corresponding to the shape of the dentition and gums of the user. Soft part 120 may be disposed so as to cover all of the inner wall and bottom surface of groove 110a of hard part 110 mentioned above, or may be disposed so as to cover a part of the inner wall and bottom surface of groove 110a.

In soft part 120, the ratio of the maximum thickness thereof to the minimum thickness thereof, that is, (maximum thickness of soft part 120)/(minimum thickness of soft part 120) is 5 or more, preferably 6 or more, and more preferably 7 or more. On the other hand, usually, the maximum value is preferably 30 or less, and more preferably 20 or less. When the ratio of the maximum thickness to the minimum thickness of soft part 120 falls within the range, the oral appliance of the present invention can correspond to a complex dentition shape and the like.

Here, the minimum thickness of soft part 120 is the minimum value of the thickness of soft part 120 when measured with a caliper. On the other hand, the maximum thickness of soft part 120 is the maximum value of the thickness of soft part 120 when measured in the same manner.

The maximum thickness of soft part 120 varies depending on the shape of the dentition and the like, but is usually, preferably 2,000 µm or less, and more preferably 1,000 µm or less. Particularly when the maximum thickness of soft part 120 is 1,000 µm or less, oral appliance 1100 does not become excessively large and further, the strength tends to be high. The minimum thickness of soft part 120 is preferably 0.1 mm or more.

The flexural modulus of soft part 120 is only required to be lower than the flexural modulus of hard part 110, and is preferably 50 to 3,000 MPa, more preferably 50 to 2,000 MPa, and further preferably 50 to 1,000 MPa. The flexural modulus is measured according to JIS T6501. Particularly when the flexural modulus of soft part 120 is 3,000 MPa or less, soft part 120 tends to trace the dentition and the like, in particular. On the other hand, when the flexural modulus of soft part 120 is 50 MPa or more, the strength of soft part 120 is sufficiently high.

The difference between the flexural modulus of soft part 120 and the flexural modulus of hard part 110 is preferably 50 to 1,600 MPa, more preferably 100 to 1,200 MPa, and further preferably 100 to 800 MPa. When the difference between the flexural modulus of hard part 110 and the flexural modulus of soft part 120 falls within the range, oral appliance body section 1110 tends to have a high strength and closely adhere to the dentition and the like.

Here, the tensile bond strength of soft part 120 and hard part 110 measured by a planar tensile test is preferably 5 to 1,000 MPa, more preferably 10 to 1,000 MPa, and further preferably 20 to 1,000 MPa. The upper limit value is more preferably 200 MPa. When the tensile bond strength of soft part 120 and hard part 110 falls within the range, the durability of oral appliance body section 1110 tends to be increased. The tensile bond strength between soft part 120 and hard part 110 can be increased by appropriately selecting the type of the resin constituting soft part 120 and the type of the resin constituting hard part 110. For example, the above tensile bond strength tends to be increased by selecting resins that can hydrogen bond with each other, or by selecting resins having high affinity or having the same structure. It is particularly preferable that both soft part 120 and hard part 110 be acrylic resins.

Here, the material of soft part 120 is not particularly limited, as long as being a resin that can be formed by the method described below, that is, formed by bringing a liquid curable resin composition into contact with a model and curing, and the resin may be a resin cured by photopolymerization, a resin cured by thermal polymerization, or the like.

Examples of the material of soft part 120 include the (meth)acrylic resin including a structural unit derived from the (meth)acrylic monomer described in hard part 110 mentioned above, a polyester resin, a polyamide resin, and polyolefin.

### (Positioning member)

The positioning member 1160 is a member for adjusting a relative position of upper jaw oral appliance body section 1111 and lower jaw oral appliance body section 1112 mentioned above and preventing the backward displacement of lower jaw oral appliance body section 1112. Positioning member 1160 in the present embodiment has upper holding section 1130 attached to the outer surface of upper jaw oral appliance body section 1111 (hard part 110), lower holding section 1140 attached to the outer surface of lower jaw oral appliance body section 1112 (hard part 110), and connecting section 1150 connecting these oral appliance bodies.

Upper holding section 1130 is not particularly limited, as long as being a structure capable of being joined to oral appliance body section 1110 (in particular, hard part 110) and capable of holding connecting section 1150 described below. For example, as illustrated in FIG. 1, upper holding section 1130 may be a structure having upper shaft body 1132 extending in an outward direction from oral appliance body section 1110, and upper flange section 1134 positioning further in an outward direction from upper shaft body 1132. One end of cylindrical upper shaft body 1132 is supported by upper jaw oral appliance body section 1111. The method for joining upper jaw oral appliance body section 1111 and upper shaft body 1132 is not particularly limited, and for example, upper shaft body 1132 may be supported by being inserted into a through hole provided in upper jaw oral appliance body section 1111, or upper shaft body 1132 may be embedded in upper jaw oral appliance body section 1111.

Also, lower holding section 1140 is not particularly limited, as long as being a structure capable of being joined to lower jaw oral appliance body section 1112 mentioned above (in particular, hard part 110) and capable of holding connecting section 1150 described below. For example, as illustrated in FIG. 1, lower holding section 1140 may be a structure having lower shaft body 1142 extending in an outward direction from lower jaw oral appliance body section 1112, and lower flange section 1144 positioning further in an outward direction from lower shaft body 1142. One end of cylindrical lower shaft body 1142 is supported by lower jaw oral appliance body section 1112. The method for joining upper jaw oral appliance body section 1112 and lower shaft body section 1142 is the same as the method for joining upper jaw oral appliance body section 1111 and upper shaft body 1132.

On the other hand, the structure of connecting section 1150 is not particularly limited, as long as it can connect upper jaw oral appliance body section 1111 and lower jaw oral appliance body section 1112 and can hold the distance between upper jaw oral appliance body section 1111 and lower jaw oral appliance body section 1112 constant. In positioning member 1160 illustrated in FIG. 1, connecting section 1150 has cylindrical outer 1152 one end of which is supported by upper shaft body 1132, inner 1154 one end of which is supported by lower shaft body 1142 and another end of which is slidably accommodated in outer 1152, and a stopper (not shown) disposed inside outer 1152, serving to determine the extension width of inner 1154. In connecting section 1150, inner 1154 is slidably accommodated relative to outer 1152, having an extendable structure. In addition, the distance and positional relationship between upper jaw oral appliance body section 1111 and lower jaw oral appliance body section 1112 can be adjusted by the position of the stopper.

Here, in positioning member 1160 of the present embodiment, lower jaw oral appliance body section 1112 is more forwardly disposed than upper jaw oral appliance body section 1111. That is, positioning member 1160 takes a structure in which lower jaw oral appliance body section 1112 is pushed from the rear to the front, and thus the backward displacement of lower jaw oral appliance body section 1112 is restricted. On the other hand, since inner 1154 is drawable from outer 1152 in a certain range, the movement of the lower jaw is hardly inhibited and the sense of incongruity of the user tends to be reduced. The restriction position and the degree of sliding of the lower jaw is adjusted by the position of the stopper (not shown) in connecting section 1150. The structure of connecting section 1150 is not limited to the above structure, and for example, connecting section 1150 may have a multi-stage extendable structure.

Positioning member 1160 described above may be made of the same material or may be made of different materials. Examples of the material of positioning member 1160 include metal materials such as titanium, iron (such as steel including stainless steel), gold, silver, platinum, cobalt, chrome, and alloys thereof, and resin materials (for example, polycarbonate resins) having a flexural modulus measured according to JIS T6501 of 1,000 MPa or more and 3,000 MPa or less. The above metal materials may have increased corrosion resistance by chemical conversion coating or the like.

### (Method for Manufacturing Oral Appliance)

The method for manufacturing oral appliance 1100 of the embodiment mentioned above will be described. The oral appliance mentioned above can be manufactured by a method including preparing a hard part having a groove for accommodating a dentition or gums (hard part preparation step), injecting a curable resin composition into the groove of the hard part (curable resin composition injection step), disposing a model having a shape of a dentition and/or gums in the groove of the hard part (model disposition step), and forming a soft part containing a material having a flexural modulus lower than that of the hard part by curing the curable resin composition in a state where the curable resin composition and the model are in contact with each other (soft part formation step). Either of the model disposition step and the curable resin composition injection step may be performed first. The method for manufacturing an oral appliance of the present invention may include steps other than the above steps, and for example, may have a positioning member attachment step of attaching a positioning member and the like.

### • Hard Part Preparation Step

The hard part preparation step is a step of preparing a hard part having a groove for accommodating a dentition or gums. In the step, the method is not particularly limited, as long as the hard part having the above-mentioned structure can be formed. For example, the hard part may be produced by heat forming (for example, suction forming, pressure forming, and suction + heat forming), by pouring a thermoplastic resin composition, a highly elastic material, or the like into a form, or by laser processing. The hard part may also be produced by milling or 3D printing techniques (stereolithography). In particular, according to stereolithography, a highly precise hard part can be produced.

Hereinafter, a method for producing the hard part by stereolithography will be described, but the method for producing the hard part is not limited to the following method. The method for producing the hard part by stereolithography includes producing a pre-hard part having an area including a prepolymer (pre-hard part production step), closely adhering a desired model to the prepolymer of the pre-hard part (model close adhesion step), and curing the prepolymer in a state where the prepolymer and the model are closely adhered to each other (curing step). As used herein, the prepolymer refers to a prepolymer in a state where the photopolymerizable component is not completely cured and has a certain degree of plasticity. When the hard part is produced by this method, the fabrication accuracy of the hard part can be increased.

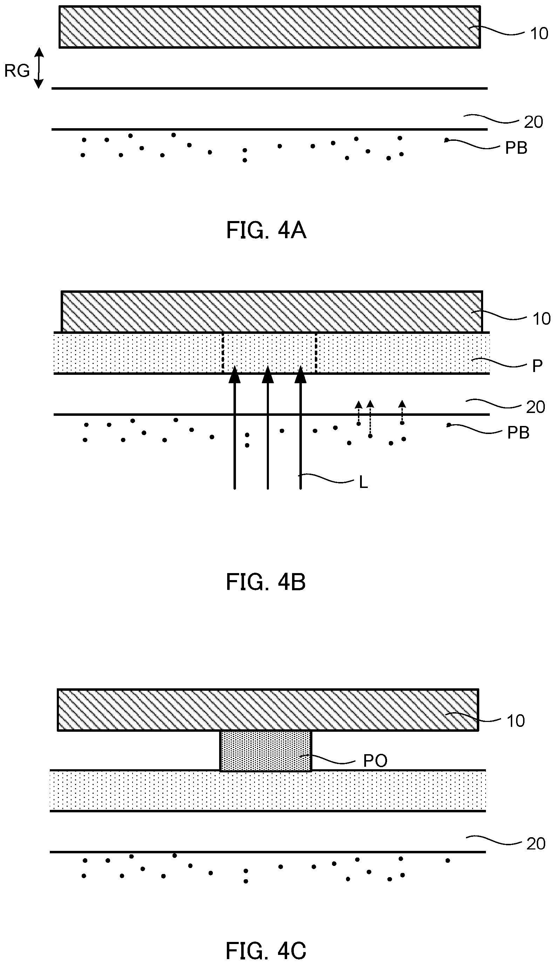

First, the pre-hard part production step will be described using FIGS. 4A to 4C. As illustrated in FIG. 4A, carrier member 10 and light transmitting member 20 are prepared in the pre-hard part formation step. Carrier member 10 is a member for raising the polymer and/or prepolymer generated by photocuring of photopolymerizable component P described below.

Here, the shape of carrier member 10 is not particularly limited, as long as carrier member 10 has an affinity with a cured product (polymer and/or prepolymer) of photopolymerizable component P and can lift the cured product. In FIG. 4A, carrier member 10 exhibits a plate-like structure, but is not limited to the structure. Carrier member 10 is controlled so as to be movable in the direction substantially perpendicular to the interface between photopolymerizable component P described below and light transmitting member 20 by a known means (not shown).

On the other hand, the structure of light transmitting member 20 is not particularly limited, as long as it can hold a desired thickness of photopolymerizable component P on one surface thereof, and for example, the structure thereof may be a dish-like or vessel-like structure.

The material of light transmitting member 20 is not particularly limited, as long as it has permeability to light L for curing photopolymerizable component P and can permeate polymerization inhibitor PB (for example, oxygen). Examples thereof include a semipermeable fluoropolymer, a crosslinked silicone polymer film, a rigid gas permeable polymer, porous glass, and combinations thereof. Examples of the semipermeable fluoropolymer include a fluoropolymer film such as an amorphous thermoplastic fluoropolymer like TEFLON (R) AF 1600 fluoropolymer film or TEFLON(R) AF 2400 fluoropolymer film; a perfluoropolyether (PFPE) film such as crosslinked perfluoropolyether film. Examples of the rigid gas permeable polymer include polymers used in the manufacture of gas permeable contact lenses. As porous glass, known porous glass and microporous glass are applicable.

The thickness of light transmitting member 20 is appropriately selected depending on the strength and the permeability of polymerization inhibitor PB, and is usually, preferably 0.1 mm to 100 mm or less, and more preferably 1 mm to 10 mm. When oxygen is used as polymerization inhibitor PB, it is preferable that light transmitting member 20 have a sufficiently high permeability of oxygen, and the permeability of oxygen of light transmitting member 20 is preferably 7.5 × 10⁻¹⁷ m²s⁻¹Pa⁻¹ (10 Barrer) or more.

Then, as illustrated in FIG. 4A and FIG. 4B, carrier member 10 and light transmitting member 20 are disposed with a predetermined space RG therebetween, and photopolymerizable component P is introduced into the gap. The method for introducing photopolymerizable component P is not particularly limited, and may be injected using a syringe (not shown) or the like.

Here, photopolymerizable component P is only required to be a liquid composition capable of being cured with light irradiation described below, and can be a composition containing a (meth)acrylic resin precursor described in the above-mentioned hard part (for example, a (meth)acrylic monomer or an oligomer thereof), and a catalyst, a polymerization initiator, or the like.

Then, as illustrated in FIG. 4B, photopolymerizable component P disposed between carrier member 10 and light transmitting member 20 is selectively irradiated with light L, and photopolymerizable component P of the portion irradiated with light L is cured. Examples of the method for selectively irradiating photopolymerizable component P with light L include digital light processing (DLP), stereolithography (SLA)), and maskless photolithography. The wavelength of light L to be irradiated is only required to be a wavelength capable of curing photopolymerizable component P (for example, ultraviolet wavelength), and is usually, appropriately selected in accordance with the absorption wavelength of the polymerization initiator contained in photopolymerizable component P and the like.

At this time, polymerization inhibitor PB is preferably introduced at the interface between photopolymerizable component P and light transmitting member 20. The method for introducing polymerization inhibitor PB is appropriately selected depending on the type of polymerization inhibitor PB, the type of light transmitting member 20, and the like. As an example, a method in which gaseous polymerization inhibitor PB (for example, oxygen) is passed through light transmitting member 20 and introduced at the interface between photopolymerizable component P and light transmitting member 20 is exemplified. By supplying polymerization inhibitor PB to the interface therebetween, cured product PO of the photopolymerizable component can be prevented from sticking to light transmitting member 20.

As illustrated in FIG. 4C, after irradiation with a sufficient amount of light L is carried out, carrier member 10 is moved away from light transmitting member 20 to lift the cured product of photopolymerizable component PO. Subsequently, photopolymerizable component P is introduced between cured product PO and light transmitting member 20 again, a desired position is irradiated with light L, and then carrier member 10 (cured product PO) is further moved away from light transmitting member 20. Carrier member 10 may be continuously raised, or may be raised in a stepwise manner. By repeating these steps, a pre-hard part in which a structure other than the groove is formed is produced.

When irradiation with light L is carried out, the amount light L irradiated is adjusted so that the area in which a groove is formed can include the prepolymer. The method for adjusting the amount light L irradiated is not particularly limited, and the amount of light L may be changed or the raising speed of carrier member 10 may be adjusted. When the amount of light L is reduced or the raising speed of carrier member 10 is increased, the amount light L irradiated is reduced and the prepolymer is easily formed.

The flexural modulus of the prepolymer is preferably 50 MPa or more and 2,000 MPa or less. Within the range, the prepolymer is easily deformed depending on the model. The flexural modulus of the prepolymer is more preferably less than 2,000 MPa, further preferably 1,500 MP or less, and particularly preferably 1,200 MPa or less. The elastic modulus of the prepolymer is more preferably more than 50 MPa, further preferably 100 MPa or more, and particularly preferably 500 MPa or more. The flexural modulus of the prepolymer is determined by storing the pre-hard part in a thermostat water bath at 37±1°C for 50±2 hours, and then measuring the flexural modulus of the prepolymer area according to ISO 20795-1:2008 (or JIS T6501:2012).

After the above pre-hard part is formed, a model having a shape corresponding to the groove is allowed to closely adhere to the area including the prepolymer (the area where the groove is formed) (model adhesion step). The shape of the model preferably corresponds to at least a part of the shape of the oral cavity. The material of the model is not particularly limited, and may be plaster, metal, or the like. As mentioned above, photopolymerizable component P is not completely cured in the area where the groove of the pre-hard part is formed, and the prepolymer can be deformed along the shape of the model.

Then, the prepolymer is cured in a state where the area including the prepolymer of the pre-hard part and the model are closely adhered to each other (curing step). The method for curing the prepolymer is not particularly limited, and may be further irradiation with light, heating and pressurization, or addition of a polymerization promoter. As a result of the series of steps described above, hard part 110 having groove 110a mentioned above is produced.

### • Curable Resin Composition Injection Step

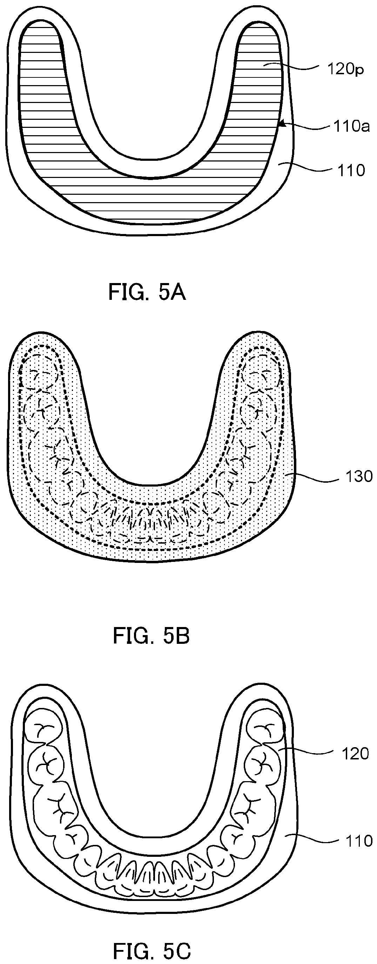

As illustrated in FIG. 5A, after preparation of hard part 110, liquid curable resin composition (precursor of the soft part) 120p is injected into the groove 110a of hard part 110 (FIG. 5A). Curable resin composition 120p is only required to be the liquid composition containing a (meth)acrylic resin precursor described in the above-mentioned soft part (for example, a (meth)acrylic monomer or an oligomer thereof) and may contain a catalyst, a polymerization initiator, or the like, as needed.

The viscosity of curable resin composition 120p measured by an E-type viscometer at 25°C is preferably 10 to 8,000 mPa.s, and more preferably 50 to 5,000 mPa.s. When the viscosity of curable resin composition 120p is within the range, the fluidity of curable resin composition 120p is high, so that curable resin composition 120p easily spreads over the entire groove of hard part 110. In addition, curable resin composition 120p easily flows along the shape of the model described below.

The method for injecting curable resin composition 120p into groove 110a of hard part 110 is not particularly limited, and examples thereof include an injection method using a syringe, a dispenser, or the like. The amount of curable resin composition 120p to be injected is appropriately selected depending on the thickness of the desired soft layer 120.

### • Model Disposition Step

After the above curable resin composition injection step, before the curable resin composition injection step, or at the same time as the curable resin composition injection step, a model disposition step of disposing model 130 having a shape corresponding to the shape of the dentition and/or gums of the user in groove 110a of hard part 110 is performed, as illustrated in FIG. 5B. The material of model 130 is not particularly limited, and may be plaster, metal, or the like. A model releasing agent, or the like may be applied to the model, as needed.

When the model disposition step is performed after the curable resin composition injection step, model 130 is disposed so as not to include air and the like between curable resin composition 120p and model 130.

### • Soft Part Formation Step

Subsequently, curable resin composition 120p is cured in a state where curable resin composition 120p and model 130 described above are in contact with each other to form soft part 120. The method for curing curable resin composition 120p is appropriately selected depending on the type of the curable resin composition.

For example, when the curable resin composition 120p is thermosetting, the curable resin composition is cured by heating. The heating time and the heating temperature are appropriately selected depending on the type of the curable resin composition 120p.

On the other hand, when the curable resin composition 120p is photocurable, the curable resin composition is irradiated with light having a desired wavelength and cured. The wavelength of the light to be irradiated is only required to be a wavelength capable of curing curable resin composition 120p (for example, ultraviolet wavelength), and is usually, appropriately selected in accordance with the absorption wavelength of the polymerization initiator contained in curable resin composition 120p, and the like. When the curable resin composition 120p is photocured, either model 130 or hard part 110 preferably has light permeability. This enables sufficient curing of curable resin composition 120p.

Then, by removing model 130, an oral appliance body section having hard part 110 and soft part 120 can be obtained. When a formed product having a relatively low hardness like soft part 120 is formed by adhering a liquid curable resin composition and a model, usually, the formed article is hardly peeled off from the model and damage may be caused in the formed article. In contrast, when hard part 110 is produced first and then soft part 120 is produced on hard part 110 like the present embodiment, soft part 120 is sufficiently supported by hard part 110, so that soft part 120 can be easily peeled from model 130. Thus, according to the above-mentioned method, the soft part sufficiently corresponding to the shape of the dentition and the like of a user can be accurately formed.

### • Positioning Member Attachment Step

On the other hand, in the positioning member attachment step, positioning member 1160 is attached to upper jaw oral appliance body section 1111 and lower jaw oral appliance body section 1112 having hard part 110 and soft part 120 obtained by the above-mentioned method. Positioning member 1160 can be produced by a known metal processing method or resin processing method.

The method for attaching positioning member 1160 to upper jaw oral appliance body section 1111 and lower jaw oral appliance body section 1112 is not particularly limited, and is appropriately selected in accordance with the shape and the like of positioning member 1160. For example, upper shaft body 1132 and lower shaft body 1142 may be attached by being inserted into a through hole provided on the above-mentioned oral appliance body section 1110. On the other hand, upper shaft body 1132 and lower shaft body 1142 may be embedded in oral appliance body section 1110 by performing the above-mentioned hard part formation step in a state where upper shaft body 1132 and lower shaft body 1142 are disposed in a predetermined position.

### 2. Modified Example

The method described in the above-mentioned hard part preparation step can be applied to conveniently produce an oral appliance body section consisting of a single layer (which does not have either one of the hard part and the soft part mentioned above). Specifically, the oral appliance body section may be produced by producing a pre-body section having an area including a prepolymer by stereolithography (pre-body section production step), closely adhering a model having a shape of a dentition and/or gums of a user to the prepolymer of the pre-body section (model adhesion step), and curing the prepolymer (curing step).

The pre-body section production step includes, as mentioned above, preparing a carrier member and a light transmitting member, disposing a photopolymerizable component between the carrier member and the light transmitting member (hereinafter, also referred to as "photopolymerizable component disposition step"), irradiating the photopolymerizable component between the carrier member and the light transmitting member with light through the light transmitting member (hereinafter, also referred to as "light irradiation step"), and moving a cured product of the carrier member and the photopolymerizable component away from the light transmitting member (hereinafter, also referred to as "carrier member movement step"). Then, the photopolymerizable component disposition step, the light irradiation step, and the carrier member movement step are repeated as needed, so that the pre-body section having a desired shape can be obtained.

In the pre-body section production step, the amount of light irradiated is adjusted in the above light irradiation step so that the prepolymer can be included in the area to which a model is allowed to closely adhere in the above-mentioned model adhesion step (the area that is formed into the shape of the dentition and gums).

Here, when a plurality of light irradiation steps is performed in the above pre-body section production step, the intensity of the light in each light irradiation step may be adjusted to partially adjust the elastic modulus and the like of the pre-body section to be produced and also, the oral appliance body section. For example, the step of irradiating a partial area of the photopolymerizable component with light having a first intensity and the step of irradiating another area of the photopolymerizable component with light having a second intensity (an intensity different from the first intensity) may be performed. In this case, the area including the prepolymer mentioned above may be formed in the step of irradiating with light having the first intensity, and the area including the prepolymer mentioned above may be formed in the step of irradiating with light having the second intensity. Thus, by performing a plurality of light irradiation steps each having a different amount of light irradiated, the elastic modulus of the pre-body section (or oral appliance body section) to be obtained can be partially adjusted. Further, light irradiation steps each having a different intensity may be performed so that the elastic modulus of the pre-body section (or oral appliance body section) can be changed by three stages or more. Alternatively, an area irradiated with light having the first intensity and an area irradiated with light having the second intensity may be provided in one light irradiation step. In this case, the areas having a different elastic modulus can be formed by a single light irradiation.

Further, when a plurality of carrier member movement steps are performed in the above-mentioned pre-body section production step, the raising speed of the carrier member in each carrier member movement step may be changed to partially adjust the elastic modulus and the like of the pre-body section and also, the oral appliance body section. For example, the step of moving the carrier member at a first speed and the step of moving the carrier member at a second speed (a speed different from the first speed) may be performed. The amount of the photopolymerizable component supplied in the photopolymerizable component disposition step performed after the carrier member movement step is different between the case where the carrier member is moved at the first speed and the case where the carrier member is moved at the second speed. When the amount of the photopolymerizable component is large, that is, when the moving speed of the carrier is fast, the elastic modulus of the cured product obtained by irradiation with light having a predetermined amount of light is low. On the other hand, when the carrier speed is slow, the elastic modulus of the cured product obtained by irradiation with light having a predetermined amount of light is high. That is, also in this case, the elastic modulus of the pre-body section (or oral appliance body section) to be obtained can be partially adjusted.

The pre-body section and also oral appliance body section having a desired elastic modulus may be produced by appropriately adjusting the amount of light irradiated in the light irradiation step and the moving speed of the carrier in the carrier member movement step described above.

Here, the flexural modulus of the area including the prepolymer produced in the above pre-body section production step is preferably 50 MPa or more and 2,000 MPa or less. Within the range, the prepolymer is easily deformed depending on the model. The flexural modulus of the prepolymer is more preferably less than 2,000 MPa, further preferably 1,500 MP or less, and particularly preferably 1,200 MPa or less. The elastic modulus of the prepolymer is more preferably more than 50 MPa, further preferably 100 MPa or more, and particularly preferably 500 MPa or more. The method for measuring the flexural modulus of the prepolymer is the same as the above-mentioned method.

According to the method, the oral appliance body section can be produced without performing the curable resin composition injection step, the model disposition step, and the soft part formation step. In addition, the material of the oral appliance body section (photopolymerizable component) used in the method may be the same as the photopolymerizable component for producing the hard part or soft part mentioned above.

### Examples

### [Reference Example 1]

Using a dental duplicating impression material (manufactured by YAMAHACHI DENTAL MFG., CO., Correcsil, dental duplicating impression) and a super hard plaster (manufactured by GC Corporation, super hard plaster, FUJIROCK), a dentition model (manufactured by Nissin Dental Products INC., INVICTUS, standard G model) was duplicated. Then, the undercut of the teeth was blocked out with the super hard plaster (manufactured by GC Corporation, super hard plaster, FUJIROCK) (model A). Paraffin wax (manufactured by QUEST CORPORATION, pure paraffin wax) was applied to model A so as to cover up to 2 mm above the tooth neck part with a thickness of approximately 1.4 mm (appliance A), thereby producing an oral appliance. At this time, paraffin wax was applied to cover the front teeth part in consideration of the undercut. Appliance A was subjected to impression taking together with the plaster model using the dental duplicating impression material (manufactured by YAMAHACHI DENTAL MFG., CO., Correcsil, dental duplicating impression), and these were used with a scan wax (manufactured by i-Cast INC., BEGO Scan Wax) to duplicate appliance A (appliance B). From appliance B thus obtained, polygon data (STL data) of appliance B was produced using a dental 3D scanner (manufactured by 3Shape A/S, ortho analyzer).

The obtained STL data was modeled by using a 3D printer (manufactured by KULZER, cara Print 4.0) and software for 3D printer (manufactured by KULZER, cara Print CAM) and setting a (methacrylic) ink for Surgical Guide manufactured by NextDent as the raw material with a pitch width of 50 µm and a support density of 70%. The support pin was set at a position that does not affect the dentition surface. The modeling angle was set in a direction that does not affect the platform of the 3D printer and the dentition surface, thereby printing a three-dimensional pre-body. At this time, the irradiation light by the 3D printer or the moving speed was adjusted so that an area of the three-dimensional pre-body (the area to be closely adhered to model A described below) could be the prepolymer. After modeling, the modeled product (three-dimensional pre-body) was taken out using a plastic spatula and cleaned using 2-propanol as a solvent by an ultrasonic cleaning machine (manufactured by Sun Election, Sun Cleaner) for 30 seconds.

The prepolymer of the cleaned modeled product (three-dimensional pre-body) was fixed on model A (sizing step), which was placed in a photopolymerization apparatus (manufactured by Heraeus, HiLite power) as it was to cure the modeled product, thereby obtaining modeled product A-1. The flexural modulus of the area containing the prepolymer before curing was 814 MPa, and the flexural modulus thereof after curing was 2535 MPa.

### [Reference Example 2]

Reference Example 2 was carried out in the same manner as Reference Example 1, except that an Ortho RIGID ink manufactured by NextDent was used as the raw material instead of a (methacrylic) ink for Surgical Guide manufactured by NextDent. Table 1 shows the values of the flexural modulus of the prepolymer before curing and the flexural modulus thereof after curing measured in the same manner as Reference Example 1.

### [Reference Example 3]

Reference Example 3 was carried out in the same manner as Reference Example 1, except that an Ortho Clear ink manufactured by NextDent was used as the raw material instead of a (methacrylic) ink for Surgical Guide manufactured by NextDent. Table 1 shows the values of the flexural modulus of the prepolymer before curing and the flexural modulus thereof after curing measured in the same manner as Reference Example 1.

### [Reference Comparative Example 1]

Reference Comparative Example 1 was carried out in the same manner as Reference Example 1, except that the cleaned modeled product was cured as it was by a photopolymerization apparatus without fixing the modeled product on model A to obtain modeled product H-1. Table 1 shows the values of the flexural modulus of the prepolymer before curing and the flexural modulus thereof after curing measured in the same manner as Reference Example 1.

### [Reference Comparative Example 2]

Reference Comparative Example 2 was carried out in the same manner as Reference Example 2, except that the cleaned modeled product was cured as it was by a photopolymerization apparatus without fixing the modeled product on model A to obtain modeled product H-2. Table 1 shows the values of the flexural modulus of the prepolymer before curing and the flexural modulus thereof after curing measured in the same manner as Reference Example 2.

### [Reference Comparative Example 3]

Using a dental duplicating impression material (manufactured by YAMAHACHI DENTAL MFG., CO., Correcsil, dental duplicating impression) and a super hard plaster (manufactured by GC Corporation, super hard plaster, FUJIROCK), a dentition model (manufactured by Nissin Dental Products INC., INVICTUS, standard G model) was duplicated. Then, the undercut of the teeth was blocked out with the super hard plaster (manufactured by GC Corporation, super hard plaster, FUJIROCK) (model A). Paraffin wax (manufactured by QUEST CORPORATION, pure paraffin wax) was applied to model A so as to cover up to 2 mm above the tooth neck part with a thickness of approximately 1.4 mm (appliance A), thereby producing an oral appliance. At this time, paraffin wax was applied to cover the front teeth part in consideration of the undercut. Appliance A was subjected to impression taking together with the plaster model using the dental duplicating impression material (manufactured by YAMAHACHI DENTAL MFG., CO., Correcsil, dental duplicating impression), and these were used with a scan wax (manufactured by i-Cast INC., BEGO Scan Wax) to duplicate appliance A (appliance B). From appliance B thus obtained, polygon data (STL data) of appliance B was produced using a dental 3D scanner (manufactured by 3Shape A/S, ortho analyzer).

The obtained STL data was modeled by using a 3D printer (manufactured by KULZER, cara Print 4.0) and software for 3D printer (manufactured by KULZER, cara Print CAM) and setting a (methacrylic) Ortho Clear ink manufactured by NextDent as the raw material with a pitch width of 50 µm and a support density of 70%. The support pin was set at a position that does not affect the dentition surface. The modeling angle was set in a direction that does not affect the platform of the 3D printer and the dentition surface, thereby printing a three-dimensional pre-body. At this time, the irradiation light by the 3D printer or the moving speed was adjusted so that an area of the three-dimensional pre-body (the area to be closely adhered to model A described below) could be the prepolymer. After modeling, the modeled product (three-dimensional pre-body) was taken out using a plastic spatula and cleaned using 2-propanol as a solvent by an ultrasonic cleaning machine (manufactured by Sun Election, Sun Cleaner) for 30 seconds and allowed to stand at room temperature as it was.

The prepolymer of the modeled product (three-dimensional pre-body) was fixed on model A, which was placed in a photopolymerization apparatus (manufactured by Heraeus, HiLite power) to cure the modeled product again, thereby obtaining modeled product H-3. Table 1 shows the values of the flexural modulus of the prepolymer before curing and the flexural modulus thereof after curing measured in the same manner as Reference Example 1.

### [Reference Comparative Example 4]

Using a dental duplicating impression material (manufactured by YAMAHACHI DENTAL MFG., CO., Correcsil, dental duplicating impression) and a super hard plaster (manufactured by GC Corporation, super hard plaster, FUJIROCK), a dentition model (manufactured by Nissin Dental Products INC., INVICTUS, standard G model) was duplicated. Then, the undercut of the teeth was blocked out with the super hard plaster (manufactured by GC Corporation, super hard plaster, FUJIROCK) (model A). Paraffin wax (manufactured by QUEST CORPORATION, pure paraffin wax) was applied to model A so as to cover up to 2 mm above the tooth neck part with a thickness of approximately 1.4 mm (appliance A), thereby producing an oral appliance. At this time, paraffin wax was applied to cover the front teeth part in consideration of the undercut. Appliance A was subjected to impression taking together with the plaster model using the dental duplicating impression material (manufactured by YAMAHACHI DENTAL MFG., CO., Correcsil, dental duplicating impression), and these were used with a scan wax (manufactured by i-Cast INC., BEGO Scan Wax) to duplicate appliance A (appliance B). From appliance B thus obtained, polygon data (STL data) of appliance B was produced using a dental 3D scanner (manufactured by 3Shape A/S, ortho analyzer).

The obtained STL data was modeled by using a 3D printer (manufactured by Formlabs, Form 2) and software for 3D printer (manufactured by KULZER, PreForm) and setting a (acrylic) Flexible FLGR02 ink manufactured by Formlabs as the raw material with a pitch width of 100 µm. The support pin was arbitrary set on the software and set at a position that does not affect the dentition surface. The modeling angle was set in a direction that does not affect the platform of the 3D printer and the dentition surface, thereby printing a three-dimensional pre-body. At this time, the irradiation light by the 3D printer or the moving speed was adjusted so that an area of the three-dimensional pre-body (the area to be closely adhered to model A described below) could be the prepolymer. After modeling, the modeled product (three-dimensional pre-body) was taken out using a plastic spatula and cleaned using 2-propanol as a solvent by an ultrasonic cleaning machine for 10 minutes.

The prepolymer of the cleaned modeled product (three-dimensional pre-body) was fixed on model A, which was placed in a photopolymerization apparatus (manufactured by Heraeus, HiLite power) as it was to cure the modeled product, thereby obtaining modeled product H-4.

### [Evaluation]

The compatibility between the obtained modeled product and model A of the modeled product was evaluated from the following two viewpoints.
Viewpoint 1) whether the modeled product can fit on model A without a large resistance to the dentition
Viewpoint 2) whether the detachment of the modeled product from model A is not observed, even when the modeled product fitted on model A is turned upside down

The compatibility with the dentition surface was evaluated under the following criteria.
A: The case where, with respect to viewpoint 1, the modeled product was able to fit on model A without a large resistance to the dentition, and with respect to viewpoint 2, the detachment of the modeled product from model A was not observed
B: The case where, with respect to viewpoint 1, the modeled product was able to fit on model A with a slight resistance to the dentition, and with respect to viewpoint 2, the detachment of the modeled product from model A was not observed
C: The case where, with respect to viewpoint 1, the modeled product had a large resistance to the dentition, or with respect to viewpoint 2, the detachment of the modeled product from model A was observed

### [Results]

Both modeled product A-1 (Reference Example 1) and modeled product A-2 (Reference Example 2) did not have a large resistance to the dentition, and the modeled product was able to fit on model A, with respect to viewpoint 1. Also, with respect to viewpoint 2, the detachment of the modeled product from model A was not observed for both Reference Examples.

For modeled product A-3 (Reference Example 3), the modeled product was able to fit on model A although it had a slight resistance to the dentition, with respect to viewpoint 1. With respect to viewpoint 2, the detachment of the modeled product from model A was not observed.

The flexural modulus of modeled product A-1 (Reference Example 1) and modeled product A-2 (Reference Example 2) before curing was lower than the flexural modulus of modeled product A-3 (Reference Example 3) before curing, and with respect to viewpoint 1, the resistance to the dentition in modeled product A-1 (Reference Example 1) and modeled product A-2 (Reference Example 2) was lower than that in modeled product A-3 (Reference Example 3). These results show that when the flexural modulus before curing is 2,000 MPa or less, the modeled product can be practically used without problem, and the flexural modulus before curing is preferably 1,500 MPa or less, and further preferably 1,200 MPa or less.

For modeled product H-1 (Reference Comparative Example 1), modeled product H-2 (Reference Comparative Example 2), and modeled product H-3 (Reference Comparative Example 3), they have a large resistance to the dentition and damage was observed in a part of model A, with respect to viewpoint 1, or the detachment of the modeled product from model A was observed, with respect to viewpoint 2.

Modeled product H-4 (Reference Comparative Example 4) did not have a large resistance to the dentition and the modeled product was able to fit on model A, with respect to viewpoint 1. However, with respect to viewpoint 2, when the modeled product fitted on model A was turned upside down, the detachment of the modeled product from model A was observed.

**[Table 1]**

| | Reference Example 1 | Reference Example 2 | Reference Example 3 | Reference Comparative Example 1 | Reference Comparative Example 2 | Reference Comparative Example 3 | Reference Comparative Example 4 |
|---|---|---|---|---|---|---|---|
| Raw material | NextDent Surgical Guide | NextDent Ortho RIGID | NextDent Ortho Clear | NextDent Surgical Guide | NextDent Ortho RIGID | NextDent Ortho Clear | Formlabs Flexible FLGR02 |
| Polymer type | Methacrylic | Methacrylic | Methacrylic | Methacrylic | Methacrylic | Methacrylic | Acrylic |
| Modeled product | Modeled product A-1 | Modeled product A-2 | Modeled product A-3 | Modeled product H-1 | Modeled product H-2 | Modeled product H-3 | Modeled product H-4 |
| Sizing step | Yes | Yes | Yes | No | No | Yes | Yes |
| Flexural modulus before curing (JIS T6501) | 814 MPa | 1098 MPa | 1871 MPa | 775 MPa | 1098 MPa | 2917 MPa | < 50 |
| Flexural modulus after curing (JIS T6501) | 2535 MPa | 2574 MPa | 2917 MPa | 2544 MPa | 2574 MPa | 2917 MPa | < 50 |
| Compatibility to dentition surface | A | A | B | C | C | C | C |

The present application claims priority based on Japanese Patent Application No. 2019-231821 filed on December 23, 2019. The contents described in the specification and accompanying drawings are incorporated herein by reference in its entirety.

### Industrial Applicability

The oral appliance of the present invention can restrict the backward displacement of the lower jaw of a user and is very effective for prevention and treatment of sleep apnea. In addition, the oral appliance of the present invention has a better comfort of wearing than the conventional oral appliance, and damage and deformation are hardly caused. Therefore, the oral appliance of the present invention is applicable to not only prevention and treatment of sleep apnea, but also prevention and treatment of temporomandibular disorder and prevention of bruxism.

### Reference Signs List

10 Carrier member
20 Light transmitting member
110 Hard part
110a Groove
120 Soft part
130 Model
1100 Oral appliance
1110 Oral appliance body section
1111 Upper jaw oral appliance body section
1112 Lower jaw oral appliance body section
1132 Upper shaft body section
1134 Upper flange section
1142 Lower shaft body section
1144 Lower flange section
1150 Connecting section
1152 Outer
1154 Inner
1160 Positioning member
P Photopolymerizable component
PB Polymerization inhibitor
PO Cured product
RG Space

## Claims

1. An oral appliance comprising an oral appliance body section including:
a hard part including a groove for accommodating a dentition and/or gums, and
a soft part disposed on an inner wall of the groove, the soft part comprising a material having a flexural modulus lower than that of the hard part,
wherein a ratio of a maximum thickness of the soft part to a minimum thickness of the soft part is 5 or more.

2. The oral appliance according to claim 1, wherein a tensile bond strength between the hard part and the soft part is 5 MPa to 1,000 MPa.

3. The oral appliance according to claim 1 or 2, wherein the soft part comprises a (meth)acrylic resin.

4. The oral appliance according to any one of claims 1 to 3, wherein the hard part comprises a (meth)acrylic resin.

5. The oral appliance according to any one of claims 1 to 4, wherein:
the oral appliance body section is constituted from a pair of an upper jaw oral appliance body section and a lower jaw oral appliance body section, and
the oral appliance body section further includes positioning members for restricting backward displacement of the lower jaw oral appliance body section.

6. A method for manufacturing an oral appliance comprising:
preparing a hard part including a groove for accommodating a dentition and/or gums;
injecting a curable resin composition into the groove of the hard part;
disposing a model having a shape of a dentition and/or gums in the groove of the hard part; and
forming a soft part comprising a material having a flexural modulus lower than that of the hard part by curing the curable resin composition in a state where the curable resin composition and the model are in contact with each other.

7. The method for manufacturing an oral appliance according to claim 6, wherein the curable resin composition comprises a photocurable component.

8. The method for manufacturing an oral appliance according to claim 7, wherein the photocurable component comprises a (meth)acrylic monomer.

9. The method for manufacturing an oral appliance according to any one of claims 6 to 8, wherein the preparing of the hard part comprises:
producing a pre-hard part having an area comprising a prepolymer by stereolithography;
closely adhering a model corresponding to the groove to the prepolymer of the pre-hard part; and
curing the prepolymer.
